# EUROPEAN PATENT APPLICATION

(11) **EP 1 932 508 A1**
(43) Date of publication of application: **18.06.2008**
(21) Application number: 05781582.1
(22) Date of filing: 05.09.2005
(51) Int. Cl.: A61K 8/35, A61K 8/19, A61K 8/92, A61Q 17/04

(54) **SELF-TANNING COSMETIC**

(71) Applicant: Shiseido Company, Limited, Chuo-ku Tokyo 104-8010 (JP)
(72) Inventor: TAKAKURA, Yoshihito, Yokohama-shi Kanagawa 224-8558 (JP)
(74) Representative: ter Meer, Nicolaus
(86) International application number: PCT/JP2005/016208
(87) International publication number: WO 2007/029299

(57) **Abstract**

The present invention provides a self tanning cosmetic comprising 0.2-20.0 mass% of dihydroxyacetone, 0.01-3.0 mass% of hydrophobed metal-containing powder, and a solid oil component in the amount of 5-20 times mass of said hydrophobed metal-containing powder wherein oil-based particles prepared by encapsulating said metal-containing powder with said solid oil component are dispersed in the water phase in which dihydroxyacetone is dissolved.

In the present invention, the decorative effect of the pearl agent and the long term dyeing effect of dihydroxyacetone can be combined to provide a self tanning cosmetic that has superior storage stability and gives a superior sensation during use.

## Description

### TECHNICAL FIELD

The present invention relates to a self tanning cosmetic. More specifically, it relates to a self tanning cosmetic containing dihydroxyacetone that can contain a pearl agent in a stable manner and gives a superior sensation during use.

### BACKGROUND ART

A self tanning cosmetic is a cosmetic that colors the skin. The coloring effect of a dye usually starts several hours after application, peaks in one to two days, and decays until the skin color returns to the original after one week.

A self tanning cosmetic is a cosmetic that provides the appearance of natural and healthy sun-tanned skin without exposure to harmful ultraviolet light. A self tanning cosmetic that contains a dye called dihydroxyacetone is known as having a sustained coloring effect (Patent Documents 1-5).

For body self tanning cosmetics, gel-like self tanning cosmetics are preferred from the point of view of adequate spreading and easy drying. Also, self tanning cosmetics containing a pearl agent to decorate the skin are very popular.
It is desirable for body self tanning cosmetics to give a refreshing and succulent sensation during use as do water based gel-like cosmetics.

Patent Document 1: Japanese Patent Laid-Open H7-101843 bulletin
Patent Document 2: Japanese Patent Laid-Open H7-101848 bulletin
Patent Document 3: Japanese Patent Laid-Open 2001-213747 bulletin
Patent Document 4: Japanese Patent Laid-Open 2002-338448 bulletin
Patent Document 5: Japanese Patent Laid-Open 2005-145860 bulletin

### DISCLOSURE OF INVENTION

### [Problem that the present invention aims to solve]

However, dihydroxyacetone that is contained in a self tanning cosmetic has a problem in that it decomposes over days and thus the color tone changes and an offensive odor emerges. In particular, dihydroxyacetone becomes extremely unstable when coloring agents such as inorganic pigments are added. Therefore, the manufacturing of a water based gel-like cosmetic using both dihydroxyacetone and pigments/pearl agents was very difficult.

In view of the aforementioned problem, the inventors conducted earnest research and discovered that the instability of dihydroxyacetone is prevented and a body self tanning cosmetic that gives a superior sensation during use can be provided by adding a pearl agent encapsulated with a solid oil component to the water phase containing dihydroxyacetone, thus completing the present invention.

The object of the present invention is to provide a self tanning cosmetic containing a pearl agent that has superior stability and gives a superior sensation during use.

### [Means to solve the Problem]

That is, the present invention provides a self tanning cosmetic comprising 0.2-20.0 mass% of dihydroxyacetone, 0.02-3.0 mass% of hydrophobed metal-containing powder, and a solid oil component in the amount of 5-20 times mass of said hydrophobed metal-containing powder wherein oil-based particles prepared by encapsulating said metal-containing powder with said solid oil component are dispersed in the water phase in which dihydroxyacetone is dissolved.

Also, the present invention provides the aforementioned self tanning cosmetic wherein said metal-containing powder is a pearl agent.

Furthermore, the present invention provides the aforementioned self tanning cosmetic wherein said solid oil component is melted at a high temperature, into which said metal-containing powder is dispersed to obtain the oil phase, which is added to the water phase while stirring is carried out and then rapidly cooled so that said encapsulated oil-based particles are dispersed in said water phase.

Also, the present invention provides the aforementioned self tanning cosmetic wherein said encapsulated oil-based particles contain an acrylic acid/alkyl acrylate copolymer.

Furthermore, the present invention provides the aforementioned self tanning cosmetic wherein the particle size of said encapsulated oil-based particles is 50 µm to 0.5 mm.

Also, the present invention provides the aforementioned self tanning cosmetic wherein said water phase contains a thickener.

Furthermore, the present invention provides the aforementioned self tanning cosmetic wherein said self tanning cosmetic is a body self tanning cosmetic.

### [Effects of the invention]

(1) The self tanning cosmetic of the present invention prevents the instability of dihydroxyacetone caused by a pearl agent (a type of metal-containing powder). That is, the decorative effect of the pearl agent and the long term dyeing effect of dihydroxyacetone can be combined to provide a self tanning cosmetic that has superior storage stability and gives a superior sensation during use.
(2) The self tanning cosmetic of the present invention gives an excellent sensation during use. That is, since it is a water based gel-like cosmetic, it spreads well and gives a refreshing and succulent sensation during use.
(3) The self tanning cosmetic of the present invention allows for the addition of a pearl agent to a clear base agent in a stable manner and therefore it can provide an appearance of a water based gel containing granules.
(4) The self tanning cosmetic of the present invention dries fast and therefore it allows users to wear clothing immediately following application. Therefore it is suitable for a body self tanning cosmetic.

### BEST MODE FOR CARRYING OUT THE INVENTION

### [Dihydroxyacetone]

The dihydroxyacetone used in the present invention, abbreviated as DHA, is a prior art cosmetic material (skin dye) commonly used in self tanning cosmetics. Its content in the self tanning cosmetic is required to be 0.2-20.0 mass%, preferably 1.0-15.0 mass%, of the total amount of the self tanning cosmetic. If the content is less than 0.2 mass%, then the self tanning effect cannot be obtained. If it is over 20.0 mass%, then the storage stability is degraded.
The purity of dihydroxyacetone used in the present invention is preferably 90.0 mass% or higher, more preferably 95.0 mass% or higher, and even more preferably 97.0 mass% or higher.
In the present invention, dihydroxyacetone is dissolved in the water phase.

### [Metal-containing powder encapsulated with the solid oil component]

The metal-containing powder used in the present invention is encapsulated with the solid oil component by means of a prior art method (Japanese Patent Laid-Open No. 2003-73230 bulletin, for example). Encapsulation with the solid oil component refers to a state in which the metal-containing powder is enwrapped in the oil-based particles composed of the solid oil component (it does not refers to a state in which the metal-containing powder is merely adhered to the surface of the oil-based particles). To put it simply, it refers to a state in which the metal-containing powder is included in a lipstick-like solid fat/oil of a particle size that allows dispersion in the water phase.
In addition to the solid oil component and the metal-containing powder, other oil components and other hydrophobic components can be contained in the oil-based particles.
The oil-based particles are dispersed in the water phase; their average particle size is preferably 50 µm to 0.5 mm.

The mass of the solid oil component for encapsulating the metal-containing powder in the self tanning cosmetic is 5 times or more and 20 times or less the mass of the hydrophobed metal-containing powder. That is, the mass ratio of the solid oil component to the hydrophobed metal-containing powder is 5/1-20/1. If the mass ratio of the solid oil component to the hydrophobed metal-containing powder is less than 5/1, then encapsulation of the metal-containing powder becomes incomplete and the storage stability becomes poor. As the solid oil component content increases, the water based gel-like sensation during use decreases; therefore the ratio of the solid oil component to the metal-containing powder is preferably small from the point of view of the sensation during use; the content in the self tanning cosmetic is also preferably 30 mass% or less.

The hydrophobed metal-containing powder content in the self tanning cosmetic is required to be 0.02-3.0 mass%, preferably 0.1-2.0 mass%.
If the content is less than 0.01 mass%, then the decorative effect on the skin and the base agent become poor. On the other hand, if it is over 3.0 mass%, then the increase in the amount of the oil component required for encapsulation results in an oily sensation during use and the water based gel-like sensation during use is lost.

In the present invention, metal-containing powder refers to powder of a metal atom chemical compound. Examples include inorganic white pigments such as titanium dioxide and zinc oxide; inorganic red pigments such as iron oxide (red iron oxide) and iron titanate; inorganic black pigments such as γ-iron oxide; inorganic purple pigments such as manganese violet and cobalt violet; inorganic green pigments such as chromium oxide, chromium hydroxide, and cobalt titanate; inorganic blue pigments such as ultramarine blue and Berlin blue; pearl pigments such as titania-coated mica, iron oxide-coated mica, titania-coated bismuth oxychloride, titania-coated talc, colored titania-coated mica, bismuth oxychloride, and fish scale flakes; and metal powder pigments such as aluminium powder and copper powder.

In terms of the decorative effect on the skin and the base agent, pearl agents are preferable for the metal-containing powder. One, two, or more types of pearl agents can be used. A pearl agent refers to powder that has interference colors; examples include titanated mica, titania-coated synthetic mica, titania- and silicon oxide-coated mica, titania-coated glass flakes, titania-coated silica flakes, titania-coated alumina flakes, titania-flakes, and silica-coated aluminum.

The surface of the metal-containing powder such as a pearl agent must be hydrophobed. The hydrophobing treatment improves the efficiency of encapsulation in the solid oil component.
Selection of the hydrophobing treatment method is not limited in particular; a prior art method is used for the treatment. Examples include a treatment in which silicones such as methylhydrogen polysiloxane, methylhydrogen polysiloxane/dimethyl polysiloxane copolymer, and dimethyl polysiloxane are used, a treatment in which silane compounds such as octyltriethoxysilane and hexyltrimethoxysilane are used, a treatment in which a fatty acid such as palmitic acid or stearic acid is used, a metal soap treatment in which an alkali metal salt or alkali earth metal salt of said fatty acid is used, and a fluorine treatment in which diethanolamine perfluoroalkylphosphate, perfluoroalkyltrimethoxysilane, etc. are used. In the present invention, a pearl agent treated with dimethyl polysiloxane is particularly preferable.

### [Preferable optional ingredients in the oil-base particles]

In order to suppress aggregation of the oil-based capsules, it is particularly preferable to blend an acrylic acid/alkyl 1 acrylate (C10-30) copolymer (PEMULEN TR-2 from BF Goodrich) in the oil-based particles composed of the solid oil component.

The content of the acrylic acid/alkyl acrylate (C10-30) copolymer in the self tanning cosmetic is required to be 0.02-4.0 mass%, preferably 0.3-0.4 mass%, of the total amount of the oil component constituting the oil-based particles in the self tanning cosmetic. If the content is less than 0.02 mass% of the total amount of the oil component, then the particle formation capability of the oil-based particles becomes insufficient and the oil-based particles aggregate, which makes encapsulation difficult. If it is over 4.0 mass%, then the thickening effect on the water phase becomes excessive and encapsulating becomes difficult.

### [Thickener]

It is preferable to add a thickener to the water phase of the self tanning cosmetic of the present invention. The selection is not limited as long as a thickener commonly used in cosmetics is selected.
However, the thickener consisting of microgel disclosed in Japanese Patent Laid-Open No. 2005-145860 bulletin is preferable considering the fact that it would coexist well with dihydroxyacetone in the water phase, it can provide a clear appearance, and it allows for a high alcohol content. Details are described below.

"The thickener consisting of a microgel" that is preferably used in the present invention is a thickener consisting of a synthetic polymer microgel obtained by using the reverse phase emulsification polymerization method for radical polymerization of water soluble ethylene-type unsaturated monomers dissolved in the dispersion phase in a composition having an organic solvent or an oil component as the dispersion medium and water as the dispersion phase. A "microgel" is synthetic polymer electrolyte fine particles manufactured by means of the reverse phase microemulsion polymerization method. The thickener consisting of the microgel of the present invention swells in water, ethanol, or a water/ethanol mixed solution to provide a highly viscous solution that appears homogeneous to the naked eye.

It is preferable to manufacture the thickener consisting of a microgel by using a surfactant whose hydrophilicity/lipophilicity balance (HLB) is specifically selected so that the reverse emulsion polymerization system forms a single phase microemulsion or fine W/0 emulsion.

For the "water soluble ethylene type unsaturated monomer", joint use of a nonionic monomer and an ionic monomer (anionic monomer or cationic monomer) is preferable.
For the nonionic monomer, the dialkylacrylamide represented by the general formula (1) is preferable.

### General formula (1)

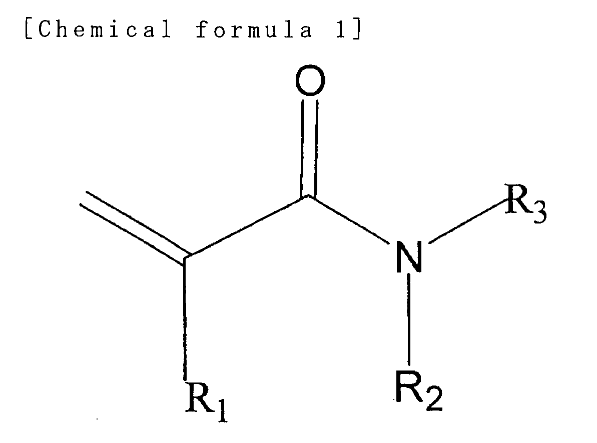

(R₁ denotes a H or methyl group; R₂ and R₃, independent of each other, denote a methyl, ethyl, propyl, or isopropyl group.)
For the "ionic monomer", the anionic acrylamide derivative represented by general formula (2) or the cationic acrylamide derivative represented by general formula (3) is preferable.

### General formula (2)

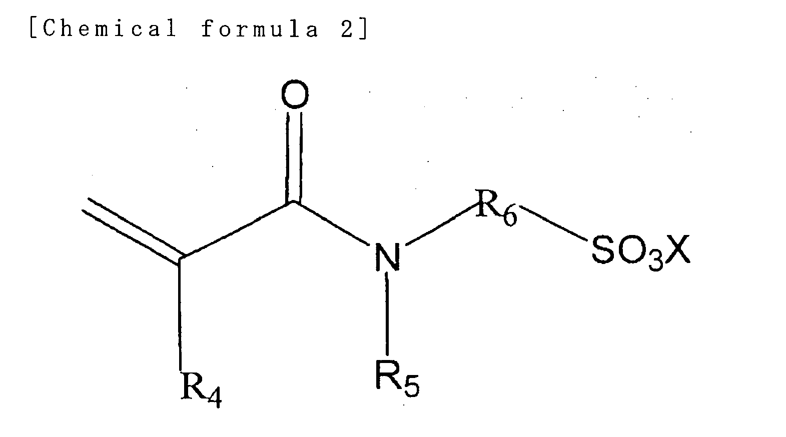

(R₄ and R₅, independent of each other, denote a H or methyl group, R₆ denotes a straight chain or branched alkyl group having 1-6 carbon atoms, and X denotes a metal ion or NH₃.)

### General formula (3)

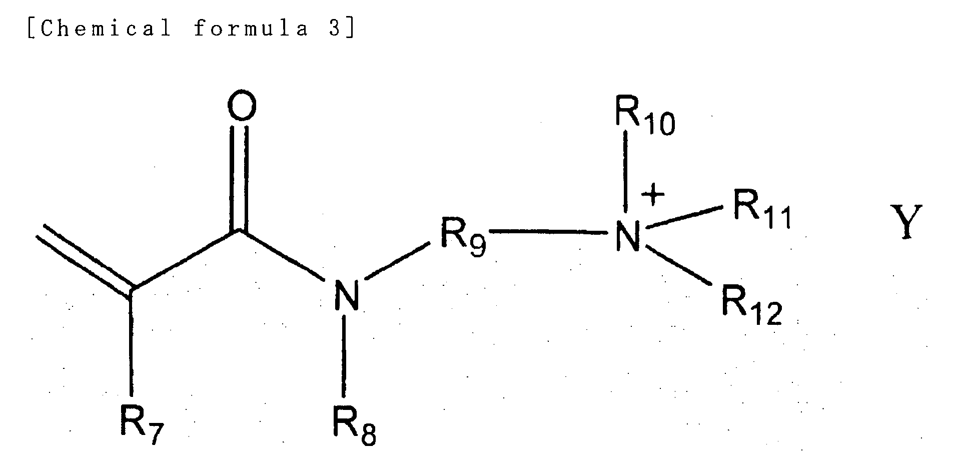

(R₇ denotes a H or methyl group, R₈ denotes a H or straight chain or branched alkyl group having 1-6 carbon atoms, R₉ denotes a straight chain or branched alkyl group having 1-6 carbon atoms, R₁₀, R₁₁, and R₁₂ denote a methyl group or ethyl group, and Y denotes a metal ion.)

Particularly preferable dialkylacrylamides are dimethylacrylamide and diethylacrylamide.
Particularly preferable ionic acrylamide derivatives are 2-acrylamide-2-methylpropanesulfonic acid and its salts.
A particularly preferable cationic acrylamide derivative is N,N-dimethylaminopropylacrylamidemethyl chloride.
The monomer composition ratio of the nonionic monomer and the ionic monomer in the polymerization system (feed ratio of the polymerization system) is selected based on the monomer composition ratio of the target microgel. The monomer composition ratio of the microgel and the feed ratio into the polymerization system are about the same. The feed ratio of the nonionic monomer and the ionic monomer in the polymerization system (molar ratio) for copolymerization is usually in the range of Nonionic monomer : Ionic monomer = 0.5 : 9.5 to 9.5 : 0.5, preferably 1 : 9 to 9 : 1, more preferably 7 : 3 to 9 : 1. The optimum ratio is Nonionic monomer : Ionic monomer = 8 : 2.
The aforementioned water soluble ethylene type unsaturated monomer is then chosen at will and the thickener is polymerized. A particularly preferable thickener is a dipolymer microgel copolymerized from monomers of dimethylacrylamide and 2-acrylamide-2-methylpropanesulfonic acid, used as the water soluble ethylene type unsaturated monomer. In this case, without requiring a cross-linking monomer, a thickener that exhibits a superior thickening effect and sensation during use can be obtained by self cross-linking. A cross-linking monomer can be used; a cross-linking monomer represented by general formula (4) is preferable, and methylenebisacrylamide is particularly preferable.

In the case of the microgel obtained by copolymerizing a dialkylacrylamide and an acrylamide type ionic monomer, a spontaneous cross-linking reaction develops and a chemically self-cross-linked microgel can be obtained without having to copolymerize with a multifunctional cross-linking monomer as a third ingredient, thus providing a particularly preferable thickener for the present invention.
Although the third ingredient, the multifunctional cross-linking monomer, is not required, the microgel used in the present invention can still be synthesized if such a monomer is added for copolymerization. For the multifunctional cross-linking monomer, the monomers represented by general formula (4) are preferable; one, two, or more of the monomers represented by general formula (4) can be used for cross-linking. It is essential that these cross-linking monomers can effectively have a cross-linking structure in the polymerization system of the dialkylacrylamide and the ionic acrylamide derivative.

### General formula (4)

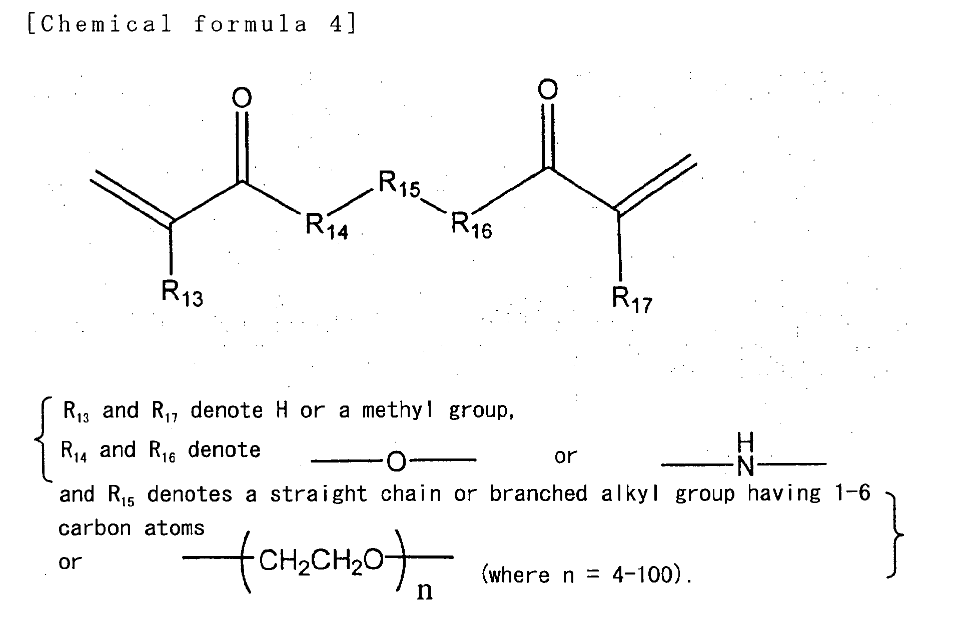

Examples of the cross-linking monomer include ethylene glycol diacrylate, ethylene glycol dimethacrylate, polyoxyethylene diacrylate, polyoxyethylene dimethacrylate, diethylene glycol dimethacrylate, trimethylolpropane triacrylate, N,N'-methylenebisacrylamide, N,N'-ethylenebisacrylamide, triallyl cyanurate, and pentaerythrithol dimethacrylate; one, two, or more chosen from these can be used. In the present invention, N,N'-methylenebisacrylamide is particularly preferable to use.
In the copolymer, the content molar ratio of the 2-acrylamide-2-methylpropanesulfonic acid unit and the dialkylacrylamide unit is usually 2-acrylamide-2-methylpropanesulfonic acid unit : dialkylacrylamide unit = 0.5 : 9.5 to 9. 5 : 0.5, preferably 1 : 9 to 9 : 1, and more preferably 3 : 7 to 1 : 9. The optimum ratio is 2-acrylamide-2-methylpropanesulfonic acid unit : dialkylacrylamide unit = 2 : 8. The viscosity of the thickener used in the present invention results from the extension of the molecular chains due to the electrostatic repulsion of the sulfonyl group, which is a strongly dissociating group, and the spontaneous cross-linking reaction of dialkylacrylamide or the cross-linked structure formed by the cross-linking monomer; if the content of the 2-acrylamide-2-methylpropanesulfonic acid unit or its salt is less than 5 mole % compared with the dialkylacrylamide unit, then a sufficient viscosity may not be obtained because a sufficient extension of the molecular chains does not occur.
The blend ratio of the cross-linking monomer is preferably 0.0001-2.0 mole % of the total moles of the 2-acrylamide-2-methylpropanesulfonic acid or its salt and the dialkylacrylamide. If it is less than 0.0001 mole %, then the obtained thickener may not exhibit the effect of cross-linking. If more than 2 mole % is used for preparation, a sufficient thickening effect may not be achieved because the cross-link density is too high and the microgel cannot swell enough.
The weight average molecular weight of the microgel is 100,000-5,000,000 (PEG equivalent, measured with the GPC); it is adjusted according to the desired viscosity of the thickener.

Following the polymerization, the microgel to be used as a thickener in the present invention can be isolated in a powder form after a precipitation/purification process.
The microgel thus isolated in a powder form is easily dispersed in water, ethanol, or a water/ethanol mixed solvent and quickly swells and functions as a thickener. It also has the advantage of providing a clear base agent.
Also, by choosing a strongly acidic monomer (a monomer containing a sulfonic acid residue, for example) for the ionic monomer to be copolymerized into the microgel, even an acidic formulation can be thickened, which was not possible with conventional carboxyvinyl polymers.
Furthermore, this microgel can thicken or gel alcohols, which conventionally was considered difficult. It manifests a particularly superior effect as a thickener for a composition containing a high concentration of alcohol.
Also, the aforementioned thickener has a thickening or gelling function in the water phase and substantially improves the dyeing ability of dihydroxyacetone. Furthermore, it allows preparation of a self tanning cosmetic that gives an exceptionally superior sensation during use.

The aforementioned thickener is added to the water phase. The blend ratio of the thickener is not limited in particular. From the usability point of view, a preferable blend ratio is 0.01-10% (mass percentage), and more preferably 0.1-5% (mass percentage), of the total amount of the self tanning cosmetic.
When ethanol is blended in the self tanning cosmetic, the ethanol content is 1-70 mass%, preferably 5-20 mass%, of the total amount of the self tanning cosmetic. However, even when the ethanol content is as high as 30 mass% or more, 30-50 mass% for example, a stable composition with a superior thickening effect can be prepared. When the blend ratio of ethanol is high, the blend ratio of the thickener is preferably a little higher, i. e. 0.01-10 mass%.

### "Specific method of manufacturing the self tanning cosmetic"

The self tanning cosmetic of the present invention is a composition prepared by dispersing oil-based particles (particles composed of a solid oil-based composition such as a lipstick, for example) in the water phase. However, the oil-based particles are large (preferable average particle size 50 µm to 0.5 mm) and visually recognizable. The sensation during use is superior, i. e. the same as that of a water-based gel containing oil-based granules.

The oil component that constitutes the oil phase has to be an oil component that is solid at ordinary temperatures (15°C) and melts at higher temperatures. For example, the following oil components can be used.
(1) Examples of the "solid fats and oils" include cacao butter, coconut oil, hydrogenated coconut oil, palm oil, palm kernel oil, Japanese core wax nucleus oil, hydrogenated oil, Japanese core wax, and hydrogenated castor oil.
(2) Examples of the "waxes" include beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, P0E hydrogenated lanolin ethyl alcohol ether, ceresin, and microcrystalline wax.

Also, the following oil components can be blended in the aforementioned solid oil component.
Examples of the "liquid fats and oils" include avocado oil, tsubaki oil, turtle fatty acid, macademia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japan gimlet oil, jojoba oil, germ oil, and triglycerin.
Examples of the "hydrocarbon oils" include liquid petrolatum, ozocerite, squalane, pristane, paraffin, squalene, and petrolatum.
Examples of the "higher fatty acids" include lauric acid, myristic acid, palmitic acid, stearic acid, behenic acid, oleic acid, undecylenic acid, isostearic acid, linolic acid, linoleic acid, eicosapentaenoic acid (EPA), and docosahexaenoic acid (DHA).
Examples of the "higher alcohols" include straight chain alcohols (for example, lauryl alcohol, cetyl alcohol, stearyl alcohol, behenyl alcohol, myristyl alcohol, oleyl alcohol, and cetostearyl alcohol) and branched chain ethyl alcohols (for example, mono stearyl glycerin ether (batyl alcohol), 2-decyltetradecynol, lanolin alcohol, cholesterol, phytosterol, hexyl dodecanol, iso stearyl alcohol, and octyl dodecanol.
Examples of the "ester oils" include isopropyl myristate, cetyl octanoate, octyl dodecyl myristate, isopropyl palmitate, butyl stearate, hexyl laurate, myristil myristate, decyl oleate, dimethyl hexyl decyl octanoate, cetyl lactate, myristil lactate, lanolin acetate, iso cetyl stearate, iso cetyl isostearate, cholesteryl hydroxy 12-stearate, di-2-ethylene glycol ethylhexanoate, dipentaerythritol fatty acid ester, n-alkylene glycol monoisostearate, neopentyl glycol dicaprate, diisostearyl malate, glyceryl di-2-heptylundecanoate, trimethylolpropane tri-2-ethylhexanoate, trimethylolpropane triisostearate, tetra-2-pentaerythritol ethylhexanoate, glycerin tri-2-ethylhexanoate, glyceryl trioctanoate, glycerin triisopalmitate, trimethylolpropane triisostearate, cetyl 2-ethyl hexanoate, 2-ethylhexyl palmitate, glycerin trimyristate, tri-2-heptyl undecanoic acid glyceride, methyl castor oil fatty acid, oleyl oleate, aceto glyceride, 2-heptyl undecyl palmitate, diisobutyl adipate, 2-octyldodecyl N-lauroyl-L-glutamate, di-2-heptyl undecyl adipate, ethyl laurate, di-2-ethylhexyl sebacate, 2-hexyl decyl myristate, 2-hexyl decyl palmitate, 2-hexyl decyl adipate, diisopropyl sebacate, 2-ethylhexyl succinate, and triethyl citrate.
Examples of the "silicone oils" include chain polysiloxanes (for example, dimethylpolysiloxane, methylphenyl polysiloxane, and diphenyl polysiloxane); ring polysiloxanes (for example, octamethylcyclotetrasiloxane, decamethyl cyclopenta siloxane, and dodecamethyl cyclohexa siloxane), silicone resins forming a three-dimensional network structure, silicone rubbers, and various modified polysiloxanes (amino-modified polysiloxane, polyether-modified polysiloxane, alkyl-modified polysiloxane, and fluorine-modified polysiloxane).

Dihydroxyacetone, i. e. the dye, is dissolved in the water phase. Water is a constituent of the water phase. The water content is usually 40-99 mass%, preferably 50-95 mass%, of the total amount of the self tanning cosmetic.

Ethanol can be blended into the water phase. It is also preferable to blend in, as a humectant, polyhydric alcohols such as glycerin and sorbitol, polysaccharides such as hyaluronic acid, dyes such as caramel, chelating agents such as edetates, stabilizers such as sodium pyrosulfite, preservatives such as methylparaben and phenoxy ethanol, amino acids such as pyrrolidone carboxylic acid, as well as other water soluble ingredients.

### <Preferable manufacturing method>

The self tanning cosmetic of the present invention is preferably prepared with the following method because it gives a cosmetic with superior storage stability over time.
(1) The solid oil component and the liquid oil components are mixed and melted at a high temperature (70-100°C, depending on the type of the oil phase). An oil soluble activator, if used, is blended in at this point.
(2) Hydrophobed metal-containing powder and an acrylic acid/alkyl acrylate copolymer (a water soluble activator in the powder form) are added thoroughly dispersed in said oil component melted at a high temperature. The dispersed mixture is used as the oil phase part.
(3) The oil phase part is gradually added to one part of water (in the amount of one time or more of the oil phase part), stirred at a high temperature (higher than the melting temperature of the oil phase), to form oil-based particles (hereafter referred to as the "A part").
(4) The A part is rapidly cooled (down to the melting temperature of the oil component or lower) as it is stirred to prepare solid oil-based particles that are encapsulated metal-containing powder particles.
(5) Hydroxyacetone and a thickener (as well as alcohol, polyhydric alcohols, and various water soluble ingredients as necessary) are dissolved in water to obtain the water phase composed of a dihydroxyacetone-containing aqueous solution.
(6) The A part is mixed into the water phase and stirred for homogenization. A water-based gel-like self tanning cosmetic containing oil-based particles (in the granular form) composed of encapsulated metal-containing powder is thus prepared.

The stirrer speed is adjusted as appropriate. The faster the speed, the finer the oil-based particles will be. When the speed is low, the oil-based particles can be made very large; however, if the speed scale is too low for the manufacturing scale, then oil separation and/or particle aggregation is induced.
An stirring apparatus with weak stirring ability such as a paddle mixer is preferable.

The particle size of the oil-based particles is required to be equal to or larger than the particle size of the metal-containing powder to be encapsulated and equal to or smaller than 2 mm. Preferable is 50 µm to 0.5 mm.
If said particle size is equal to or less than the particle size of the metal-containing powder, then instability due to contacts between the metal-containing powder and the dihydroxyacetone cannot be suppressed. Also, finer oil-based particles would decrease the transparency and therefore the water-based gel-like appearance would be lost.
If said particle size is over 2 mm, then the encapsulated granular metal-containing powder oil-based particles are hard to destroy and the metal-containing powder spreads unevenly on the skin, making it impossible to obtain a good skin decoration effect.

The liquid temperature at the time when the A part is mixed into the water phase and stirred is adjusted according to the oil component composition constituting the oil-based particles. Preferable is 15-50°C, more preferable is 20-45°C. If said liquid temperature during the stirring is within the indicated range, then a cosmetic with superior stability of dihydroxyacetone is provided.
For stirring, a common emulsifying device such as a paddle mixer, homomixer, or disper can be used. A paddle mixer with weak stirring ability is preferable.

Other ingredients can be added to the self tanning cosmetic of the present invention within a range that does not degrade the effect of the present invention. For example, humectants, water-soluble polymers, thickeners, coating agents, ultraviolet light absorbents, sequestering agents, lower alcohols, polyhydric alcohols, sugars, amino acids, organic amines, polymer emulsions, pH adjusting agents, skin nutrients, vitamins, antioxidants, antioxidation assistants, dyes, and perfumes can be blended in as necessary; and the self tanning cosmetic can be prepared with a conventional method.

### <Preferable product form>

The self tanning cosmetic of the present invention is preferably a body water-based self tanning gel having an appearance wherein the oil-based particles containing the pearl agent are visually observable (appearance that shows them in a granular form in clear gel).

### EXAMPLES

The present invention is described in detail below by referring to Examples. The present invention is not limited to these examples. The blend ratios are in mass-percentage units unless specified otherwise.

Using the recipes shown in Tables 1 to 3, water-based gel self tanning cosmetics were prepared and the following effects were investigated.

### <Evaluation of appearance and odor stability>

Each cosmetic was kept in thermostatic baths at temperatures -20°C, -5°C , -0°C , room temperature (25°C), 37°C, and 50°C ; after one month the stability in terms of appearance and odor was comprehensively evaluated using the following evaluation criteria based on visual observation of the color tone and whether or not odor was generated.

### "Evaluation criteria"

No change in color tone, no odor generation ○ Some color tone change and/or odor generation Δ Very significant color tone change and/or odor generation ×

### <Evaluation of viscosity stability>

Each cosmetic was kept in thermostatic baths at temperatures -20°C, -5°C, -0°C, room temperature (25°C), 37°C, and 50°C; after one month the stability in terms of viscosity was evaluated using the following evaluation criteria.

### "Evaluation criteria"

No significant change from the freshly prepared state ○
Only at 50°C, a significant reduction in viscosity is observed compared with the freshly prepared state Δ
At room temperature to 50°C, a significant reduction in viscosity is observed compared with the freshly prepared state ×

### <Evaluation of the sustained dyeing effect>

18 microliters each of the cosmetic was applied on a 3 x 3 cm² area on the forearms of seven test subjects and washed off after two hours; the sustained dyeing effect (long term coloring effect) was evaluated after four days by using the following evaluation criteria. "Evaluation criteria"
The dyeing effect is sustained to the degree where the application site is perfectly recognizable. ○
The application site is largely recognizable, but the boundary of the dyed site is not recognizable. Δ
The skin color is completely back to the original color and the application site is not recognizable. ×

### <Evaluation of the sensation during use>

Each cosmetic was applied on the face and the sensation at the time of application was evaluated using the following evaluation criteria. "Evaluation criteria"
The sensation during use is succulent such as that of a water-based gel, and drying is fast. ○
The sensation during use is close to that of an emulsion and drying is slow, too. ×

### <Evaluation of the decorative effect on the skin>

Each cosmetic was applied on the face and the decorative effect of the pearl agent on the skin immediately following the application was visually evaluated using the following evaluation criteria. In order to observe the interference light, the evaluation was performed under sunlight. "Evaluation criteria"
The skin color change between before and after application can be verified and the light interference effect of the pearl agent can be verified. ○
The skin color change between before and after application can be verified but unevenness of the pearl agent can be observed. Δ
No skin color change between before and after the application is verified. ×

**[Table 1]**

| | | | | Encapsulated or not Hydrophobed or not | | DHA content is out or range | | Pearl agent content is out of range (the mass ratio of the pearl agent to the solid oil component is the same as in Examples 1) | |
|---|---|---|---|---|---|---|---|---|---|
| | | | Example 1 | Compara -tive example 1 | Compara -tive example 2 | Compara -tive example 3 | Compara -tive example 4 | Compara -tive example 5 | Compara -tive example 6 |
| A | lon-exchanged water | | Balance | Balance | Balance | Balance | Balance | Balance | Balance |
| | Glycerin | | 3 | 3 | 3 | 3 | 3 | 3 | 3 |
| | Edetate | | 0.03 | 0.03 | 0.03 | 0.03 | 0.03 | 0.03 | 0.03 |
| | Sodium pyrosulfite | | 0. 02 | 0.02 | 0.02 | 0.02 | 0. 02 | 0. 02 | 0. 02 |
| | Dipropylene glycol | | 5 | 5 | 5 | 5 | 5 | 5 | 5 |
| | Thickener composed of microgel | *1 | 0.6 | 0.6 | 0.6 | 0.6 | 0.6 | 0.6 | 0.6 |
| | Dihydroxyacetone | | 5 | 5 | 5 | 0.1 | 25 | 5 | 5 |
| | Ethyl alcohol (95%) | | 4 | 4 | 4 | 4 | 4 | 4 | 4 |
| | Phenoxy ethanol | | 0. 35 | 0. 35 | 0. 35 | 0.35 | 0.35 | 0.35 | 0. 35 |
| B | lon-exchanged water | | 20 | - | - | 20 | 20 | 20 | 37 |
| C | Behenyl alcohol | | 0. 375 | - | - | 0. 375 | 0. 375 | 0.005 | 1. 55 |
| | Dimthylpolysiloxane | | 1. 85 | - | - | 1. 85 | 1.85 | 0.025 | 7.65 |
| | Methylphenylsiloxane | | 1 | - | - | 1 | 1 | 0.013 | 4. 13 |
| | Pentaerythrityl tetraoctanoate | | 3. 9 | - | - | 3. 9 | 3.9 | 0. 052 | 16.12 |
| | Candelilia a wax | | 0.975 | - | - | 0.975 | 0.975 | 0.013 | 4.03 |
| D | Dimeticone-treated titanium oxide-coated mica | *2 | 0.75 | - | - | 0.75 | 0.75 | 0. 01 | 3.1 |
| | Titanium oxide-coated mica | *3 | - | - | 0.75 | - | - | - | - |
| | (Acrylic acid/alkyl acrylate (C10-30)) copolymer | *4 | 0.03 | - | - | 0.03 | 0.03 | 0.0004 | 0.12 |
| Total | | | 100 | 100 | 100 | 100 | 100 | 100 | 100 |
| Oil based particle size | | | 0.5mm | 0.5mm | 0.5mm | 0.5mm | 0.5mm | 0.5mm | 0.5mm |
| Appearance and odor stability | | | ○ | ○ | × | ○ | × | ○ | ○ |
| Viscosity stability | | | ○ | ○ | × | ○ | × | ○ | ○ |
| Sustained dyeing effect | | | ○ | ○ | ○ | × | ○ | ○ | ○ |
| Sensation during use | | | ○ | ○ | ○ | ○ | ○ | ○ | × |
| Decorative effect on the skin | | | ○ | × | ○ | ○ | ○ | × | ○ |

**[Table 2]**

| | | | Comparison of mass ratios between oil and pearl agent | | Comparison of pemulen 4 contents | | Comparison of oil-based particle sizes | |
|---|---|---|---|---|---|---|---|---|
| | | | Compara -tive example 8 | Compara -tive example e 9 | Compara -tive example 10 | Compara -tive example 11 | Compara -tive example 12 | Compara -tive example 13 |
| A | lon-exchanged water | | Balance | Balance | Balance | Balance | Balance | Balance |
| | Glycerin | | 3 | 3 | 3 | 3 | 3 | 3 |
| | Edetate | | 0.03 | 0.03 | 0.03 | 0.03 | 0.03 | 0.03 |
| | Sodium pyrosulfite | | 0. 02 | 0. 02 | 0. 02 | 0. 02 | 0. 02 | 0. 02 |
| | Dipropylene glycol | | 5 | 5 | 5 | 5 | 5 | 5 |
| | Thickener composed of microgel | *1 | 0.6 | 0.6 | 0.6 | 0.6 | 0.6 | 0.6 |
| | Dihydroxyacetone | | 5 | 5 | 5 | 5 | 5 | 5 |
| | Ethyl alcohol (95%) | | 4 | 4 | 4 | 4 | 4 | 4 |
| | Phenoxy ethanol | | 0. 35 | 0. 35 | 0. 35 | 0. 35 | 0. 35 | 0. 35 |
| B | lon-exchanged water | | 20 | 20 | 20 | 20 | 20 | 20 |
| | C Behenyl alcohol | | 0. 375 | 0.138889 | 0. 375 | 0. 375 | 0. 375 | 0. 375 |
| | Dimethylpolysiloxane | | 1. 85 | 0.685185 | 1. 85 | 1. 85 | 1. 85 | 1. 85 |
| | Methylphenylsiloxane | | 1 | 0.37037 | 1 | 1 | 1 | 1 |
| | Pentaerythrityl tetraoctanoate | | 3. 9 | 1.444444 | 3.9 | 3. 9 | 3. 9 | 3.9 |
| | Candelilia wax | | 0. 975 | 0.361111 | 0. 975 | 0. 975 | 0. 975 | 0. 975 |
| D | Dimeticone-treated titanium ox i decoated mica | *2 | 2 | 0.75 | 0.75 | 0.75 | 0.75 | 0.75 |
| | Titanium oxide-coated mica | *3 | - | - | - | - | - | - |
| | (Acrylic acid/alkyl acrylate (C10-30)) copolymer | *4 | 0.03 | 0.03 | 0.00081 | 0.4 | 0.03 | 0.03 |
| Total | | | 100 | 100 | 100 | 100 | 100 | 100 |
| Oil based particle size | | | 0.5mm | 0.5mm | - | - | 10µm | 3mm |
| Appearance and odor stability | | | Δ | Δ | Impossible to prepare | Impossible to prepare | × | ○ |
| Viscosity stability | | | × | × | | | × | ○ |
| Sustained dyeing effect | | | ○ | ○ | | | ○ | ○ |
| Sensation during use | | | ○ | ○ | | | × | ○ |
| Decorative effect on the skin | | | ○ | ○ | | | ○ | Δ |

**[Table 3]**

| | | | Example 2 | Example 3 | Example 4 |
|---|---|---|---|---|---|
| A | lon-exchanged water | | Balance | Balance | Balance |
| | Glycerin | | 3 | 3 | 3 |
| | Edetate | | 0.03 | 0.03 | 0.03 |
| | Sodium pyrosulfite | | 0.02 | 0.02 | 0.02 |
| | Dipropylene glycol | | 5 | 5 | 5 |
| | Polyacrylmide | *5 | 0.8 | - | 0.8 |
| | Choline chloride methacrylate | *6 | - | 0.8 | - |
| | Dihydroxyacetone | | 5 | 5 | 5 |
| | Ethyl alcohol (95%) | | 4 | 4 | 4 |
| | Phenoxy ethanol | | 0. 35 | 0. 35 | 0. 35 |
| B | Ion-exchanged water | | 20 | 20 | 20 |
| C | Beheny alcohol | | 0. 375 | 0. 375 | 0. 375 |
| | Dimethylpolysiloxane | | 1.85 | 1.85 | 1.85 |
| | Methylphenylsiloxane | | 1 | 1 | 1 |
| | Pentaerythrityl tetraoctanoate | | 3. 9 | 3. 9 | 3. 9 |
| | Candelilla wax | | 0.975 | 0.975 | 0.975 |
| D | Dimeticone-treated titanium oxide-coated mica | *2 | 0.75 | 0.75 | - |
| | Alkyl-modified silicone resin-coated red iron oxide | *7 | - | - | 0.2 |
| | Alkyl-modified silicone resin-coated yellow iron oxide | *8 | - | - | 0.2 |
| | Alkyl-modified silicone resin-coated black iron oxide | *9 | - | - | 0.2 |
| | (Acrylic acid/alkyl acrylate (C10-30)) copolymer | *4 | 0.03 | 0.03 | 0.03 |
| Total | | | 100 | 100 | 100 |
| Oil based particle size | | | 0.5mm | 0.5mm | 0.5mm |
| Appearance and odor stability | | | ○ | ○ | ○ |
| Viscosity stability | | | ○ | ○ | ○ |
| Sustained dyeing effect | | | ○ | ○ | ○ |
| Sensation during use | | | ○ | ○ | ○ |
| Decorative effect on the skin | | | ○ | ○ | ○ |

| | | | | | |
|---|---|---|---|---|---|
| *1: Microgel prepared in the following Synthetic example 1. 40g of dialkylacrylamide (from Kohjin) and 9g of 2-acrylamide-2-methylpropanesulfonic acid (from Sigma) are dissolved in 250g of ion-exchanged water and the pH is adjusted to 7.0 with sodium hydroxide. 250 g of n-hexane, 8.2 g of polyoxyethylene (3) oleyl ether (EMALEX 503 from Nihon Emulsion), and 16.4 g of polyoxyethylene (6) oleyl ether (EMALEX 506 from Nihon Emulsion) are put into a 1,000 ml three-neck flask equipped with a refluxing apparatus, mixed and dissolved, followed by N2 substitution. The monomer aqueous solution is added to this three-neck flask, and the temperature is raised to 65°C-70°C using an oil bath as stirring is carried out in an N2 atmosphere. When the system temperature reaches 65°C-70°C, after confirming that the system has become a semitransparent microemulsion state, 2 g of ammonium persulfate is added to the polymerization system to start the polymerization. The temperature of the polymerization system is maintained at 65°C -70°C for three hours while stirring to obtain the microgel. After the completion of the polymerization, acetone is added to the microgel suspension to precipitate the microgel, followed by rinsing with acetone three times to remove the remaining monomers and the surfactant. The precipitate is filtered and then dried under a reduced pressure to obtain the dried microgel in a white powder form. ┘ *2: SA-Timica Golden Bronze from Miyoshi Kasei *3: Timica Golden Bronze from Maar *4: PEMULEN TR-2 from Nikko Chemicals Co. *5: Sepigel 305 from Sepic *6: Salcare SC96 from Ciba Specialty Chemicals *7: Prepared by bringing red iron oxide into vapor phase contact with 1,3,5,7-tetramethylcyclotetrasiloxane and then adding tetradecene, followed by filtration, rinsing, and drying. *8: Prepared by bringing yellow iron oxide into vapor phase contact with 1,3,5,7-tetramethylcyclotetrasiloxane and then adding tetradecene, followed by filtration, rinsing, and drying. *9: Prepared by bringing black iron oxide into vapor phase contact with 1,3,5,7-tetramethylcyclotetrasiloxane and then adding tetradecene, followed by filtration, rinsing, and drying. | | | | | |

As indicated in the aforementioned Tables 1 to 3, Examples of the present invention prevent instability of dihydroxyacetone due to the pearl agent. It is a water-based gel-like self tanning cosmetic with excellent storage stability. It also give an excellent sensation during use. That is, it gives a refreshing and succulent sensation during use like that of water-based cosmetics such as gels. Furthermore, it is a self tanning cosmetic that feels clear and has a decorative effect on the skin and the cosmetic base agent; it also dries fast after application.

### INDUSTRIAL APPLICABILITY

The self tanning cosmetic of the present invention is a cosmetic used to give a suntan color to the skin without exposure to sunlight (ultraviolet light); it is a self tanning cosmetic having an instantaneous decorative effect by the pearl agent and a long term coloring effect. It is preferably used as a body self tanning cosmetic that gives a superior sensation during use.

## Claims

1. A self tanning cosmetic comprising 0.2-20.0 mass% of dihydroxyacetone, 0.02-3.0 mass% of hydrophobed metal-containing powder, and a solid oil component in the amount of 5-20 times mass of said hydrophobed metal-containing powder wherein oil-based particles prepared by encapsulating said metal-containing powder with said solid oil component are dispersed in the water phase in which dihydroxyacetone is dissolved.

2. The self tanning cosmetic of claim 1 wherein said metal-containing powder is a pearl agent.

3. The self tanning cosmetic of claim 1 or 2 wherein said solid oil component is melted at a high temperature, into which said metal-containing powder is dispersed to obtain the oil phase, which is added to the water phase while stirring is carried out and then rapidly cooled so that said encapsulated oil-based particles are dispersed in said water phase.

4. The self tanning cosmetic of claim 1, 2, or 3 wherein said encapsulated oil-based particles contain an acrylic acid/alkyl acrylate copolymer.

5. The self tanning cosmetic of claim 1, 2, 3, or 4 wherein the particle size of said encapsulated oil-based particles is 50 µm to 0.5 mm.

6. The self tanning cosmetic of claim 1, 2, 3, 4, or 5 wherein said water phase contains a thickener.

7. The self tanning cosmetic of claim 1, 2, 3, 5, or 6 wherein said self tanning cosmetic is a body self tanning cosmetic.
